# EUROPÄISCHE PATENTANMELDUNG

(11) **EP 0 628 814 A1**
(43) Veröffentlichungstag der Anmeldung: **14.12.1994**
(21) Anmeldenummer: 93109463.5
(22) Anmeldetag: 14.06.1993
(51) Int. Cl.: G01N 31/22, A61L 2/26

(54) **Prüfkörpersystem zum Überprüfen der Luftentfernung und/oder Sterilisationskontrolle kompletter Chargen in Dampfsterilisatoren**

(71) Anmelder: Kaiser, Ulrich, Dr., D-61479 Glashütten (DE)
(72) Erfinder: Kaiser, Ulrich, Dr., D-61479 Glashütten (DE)
(74) Vertreter: Mentzel, Norbert, Dipl.-Phys.

(57) **Zusammenfassung**

Prüfkörpersystem zum Überprüfen der Luftentfernung und/oder Sterilisationskontrolle kompletter Chargen in Dampfsterilisatoren.

Der Prüfkörper (1-) besteht aus einem einen Anschlußschlauch (3-) aufweisenden Grundkörper (11) mit einem darin angeordneten Aufnahmeschlitz (24) zum Einbringen eines Chemo-Indikators (25). Das freie Ende des Grundkörpers ist mit einer Kappe (17) lösbar verschlossen.

Die bisher auf dem Markt befindlichen Prüfkörper zeigen bei längerer Sterilisationszeit Fehlinterpretationen des Chemo-Indikators, da der Wärmeübergang über die Wandungen des Prüfkörpers bei längeren Sterilisationszeiten zugeführt wird. Der beschriebene Prüfkörper minimiert die Aufheizung des Chemo-Indikators durch die Prüfkörperwand dadurch, daß einerseits für den Grundkörper (11) und die Schraubkappe (17) Materialien hoher Wärmekapazität Verwendung finden und der Grundkörper allseits von einem Isolierstoffmantel (20) umgeben ist.

## Beschreibung

Die Erfindung betrifft ein Prüfkörpersystem zum Überprüfen der Luftentfernung und/oder Sterilisationskontrolle kompletter Chargen in Dampf-Sterilisatoren, bestehend aus einem einen Anschlußschlauch aufweisenden Grundkörper mit einem darin angeordneten Aufnahmeschlitz, in dem sich ein Chemo-Indikator befindet und aus einer das freie Ende des Grundkörpers lösbar verschließenden Kappe.

Moderne fra-ktionierte Vakuum-Sterilisatoren sind in der Lage bei einer Dampf-Temperatur von 134 Grad Celsius Sterilisationen auch von schwer zu sterilisierenden Produkten innerhalb von 4 - 6 Minuten sicher zu erreichen. Diese hohe Sterilisationsgeschwindigkeit beruht auf der Tatsache, das große Wärmeenergiemengen in kürzester Zeit über die Kondensationswärme des Dampfes auf das zu sterilisierende Gut übertragen werden können. Dieser schnelle Wärmeübergang findet jedoch nur dann statt, wenn die Dampf-Kondensation durch Inertgase, wie Luft oder CO₂ , nicht behindert wird.

Selbst kleine Restluftmengen im Sterilisator konzentrieren sich in Sterilisationscontainern auf, die eine hohe Wärmekapazität zur Aufheizung benötigen, weil die dort benötigte Dampfmenge die Restluft mitführt. Diese lagert sich als GAsblase um das zu sterilisierende Gut und verhindert die weitere Kondensation von Dampf.

Das Vorhandensein von Luft oder anderen nicht kondensierbaren Gasen kann durch mangelnde Luftentfernung im vorgeschalteten fraktionieren Vakuum bedingt sein, durch Leckagen an der Türdichtung, Ventilen oder Verschraubungen im Sterilisator oder durch Inertgaszutritt zusammen mit der Dampfeinführung erfolgen. Als weitere Fehler können zu niedrige Dampftemerpatur oder zu kurze Sterilisationszeiten oder auch Regelungsfehler des Sterilisators auftreten.

Um die genügende Luftentfernung un damit Dampfdurchdringung zu testen, wurde von Bowie und Dick (Bowie, I.W., e.a., The Bowie + Dick autoclave tape test, Lancet I, 1963; 585 - 87) ein Wäschepaket eingeführt, daß zwischenzeitlich nach der DIN-Norm und neuen CEN-Entwürfen modifiziert wurde. Mit Hilfe eines 6,6 kg Wäschepaketes und eines darin zentral befindlichen Chemo-Indikatorblattes in DIN A 4 Größe können Fehler sicher detektiert werden. Der Nachteil dieses BD-Testpaketes besteht in seiner mangelnden Reproduzierbarkeit aufgrund der Wäschequalität, Wäschehistorie und individuellen Packungen. Der Packungsaufwand und die täglichen Kosten für diese Wäsche sind ebenfalls nachteilig.

Bei dem aus dem deutschen Gebrauchsmuster 87 00 471 ersichtlichen Prüfkörper ist erstmalig ein Chemo-Indikator eingesetzt worden, so daß damit ein preiswert herzustellender, einfach zu handhabender Prüfkörper schwer zu sterilisierende Bedingungen simuliert und der im Prüfkörper befindliche Chemo-Indikator je nach Einsatzzweck über bestimmte Zeit- und Temperaturzyklen einen Farbumschlag aufweist und damit indirekt entweder Luftentfernung oder Sterilität der zu prüfenden Charge nachweisen kann. Die Funktion dieser vorbekannten Einrichtung besteht darin, daß der Dampf durch den Anschlußschlauch in den Prüfkörper eindringt, wobei der Dampf durch seine Kondensationswärme den im Prüfkörper befindlichen Chemo-Indikator aufheizt und entsprechend seinem Temperatur-Zeit -Verhalten zum eingestellten Farbumschlag des Chemo-Indikators führt.

Befindet sich Luft im Sterilisator, dringt Luft auch über den Schlauch in den Prüfkörper ein. Sie sammelt sich am Ende des Prüfkörpers und verhindert so, daß der im Prüfkörper befindliche Indikator ganz o. teilweise mit dem Dampf in Kontakt kommt. Eine richtige Anzeige des Indikators erfolgt nur dann, wenn durch die Blockade des Dampfzutritts kein Farbumschlag des Indikators erfolgt ist. Der vorbekannte Prüfkörper aus Kunststoff hat jedoch den Nachteil, daß bei längeren Sterilisationszeiten als 5 Min. die Wärme nicht nur über die Dampfzuführung durch den Schlauchanschluß, sondern auch über den Prüfkörper selbst zugeführt wird. Wird der vorgenannte Prüfkörper in Sterilisationsprozessen eingesetzt, deren Haltezeit länger als 5 Min. ist, heizt sich der Chemo-Indikator im Prüfkörper über die Prüfkörperwandungen selbst auf, und führt dort zu einem Umschlagen des Chemo-Indikators, obwohl sich Luft im Sterilisator befindet.

Es werden auch Sterilisationsprozesse benötigt, die länger als 5 Min. einer Temperatur von 134 Grad Celsius ausgesetzt werden. Instrumentarien, die eine derart hohe Sterilisationstemperatur nicht aushalten, werden bei 121 Grad Celsius zwischen 15 und 30 Minuten sterilisiert, um alle patogenen Mikroorganismen abzutöten. Wird bei einem derartigen Sterilisationsprozeß der vorbekannte Prüfkörper verwendet, so erfolgt während dieses Zeitraums ein Wärmedurchgang durch die Außenwände des Prüfkörpers, so daß sich der Indikator im Prüfkörper im Regelfall nach mehr als 5 Min. Verweilzeit auch dann umfärbt, wenn sich Luft im Sterilisator befindet. Die Aufheizung des Chemo-Indikators über die Wände des Prüfkörpers verursachen dann eine Fehlinformation bei der Anzeige.

Aufgabe der Erfindung ist es, einen Prüfkörper der eingangs genannten Art dahingehend zu verbessern, daß mit diesem bei der Durchführung von Sterilisationsprozessen über 5 Min. eine Aufheizug des Chemo-Indikators über den Prüfkörper infolge Wärmeübergangs durch die Wände der Prüfkörpers vermieden wird, damit der Farbumschlag des Chemo-Indikators ausschließlich durch den Dampf selbst erfolgen kann.

Diese Aufgabe wird erfindungsgemäß dadurch gelöst, daß der Grundkörper und die Kappe für eine hohe Wärmekapazität ausgebildet und im zusammengefügten Zustand von einem Isoliermantel umgeben sind. Der Vorteil eines solchen, den Sterilisationsprozeß überwachenden Prüfkörpersystems besteht darin, daß der Wärmedurchgang durch den Prüfkörper einerseits in Abhängigkeit vom Isolationsmaterial stark reduziert wird, andererseits durch die realtiv hohe Wärmekapazität des Innenprüfkörpers selbst ein schnelles Ansteigen der Temperatur des Prüfkörpers selbst von außen vermieden wird.

Damit findet auch bei langen Sterilisationsprozessen der Farbumschlag des Chemo-Indikators nur bei Dampfzutritt durch den angeschlossenen Schlauch statt.

Der in der Erfindung aufgeführte Prüfkörper hat den Vorteil, daß er sowohl als Bowie-Dick-Simulationstest zur Luftentfernung, als auch als Chargenkontrollsystem eingesetzt werden kann. Gegenüber herkömmlichen Bowie-Dick-Simulations-Testsystemen hat er den Vorteil, daß sich der Prüfkörper selbst bei längerer Benutzung nicht verändert und damit die Prüfergebnisse über lange Zeit reproduzierbar sind. Weiterhin ersteht kein Abfall wie bei den sonst üblichen Bowie-Dick- oder Chargen-Testprüfkörpern, die aus einmal verwendeten Kunststoffen- oder Papier-paketen bestehen.

Ein weiterer Vorteil besteht darin, daß die heute verwendeten Bowie-Dick-Testsysteme nur mit einem im Sterilisator eingebauten Bowie-Dick-Testprogramm von 134 Grad Celsius und 3,5 Min. getestet werden können. Alle anderen Sterilisatoren können bisher überhaupt kein Bowie-Dick-Testprogramm aus den oben beschriebenen Gründen durchführen. Der in der Erfindung beschriebene Prüfkörper gestattet es erstmalig, einen Bowie-Dick-Test auch dort auszuführen, wo kein Bowie-Dick-Test-Prüfprogramm in den Sterilisator eingebaut ist. Dafür können normale Sterilisationsprogramme bei 134 Grad Celsius zwischen 5 und 10 Minuten verwendet werden. Diese Tatsache eröffnet allen Krankenhäusern, die im Sterilisator kein Bowie-Dick-Testprogramm eingebaut haben, die Möglichkeit, den vorgeschriebenen Bowie-Dick-Test trotzdem durchzuführen.

### Ausführungsbeispiel:

Der Grundkörper und die Schraubkappe bestehen vorteilhafterweise aus Metall. Wobei der Grundkörper mit einer fest an diesem verbundenen Isolierstoffhülse umgeben ist und auch die Kappe in fester Verbindung mit der Isolierkappe steht. Um den Wärmeübergang im Bereich der Trennfuge zwischen Grundkörper und Schraubkappe und minimieren, kann zwischen Grundkörper und Schraubkappe eine über die Wandstärken von Grundkörper und Isolierstoffhülse reichende Dichtung angeordnet sein. Dabei mag der Grundkörper vorzugsweise als Hohlzylinder mit einem einseitigen Boden ausgebildet sein, in dessen Innenraum ein mit einem Längsschlitz zur Aufnahme des Chemo-Indikators versehener Aufnahmedorn einführbar ist. Der Aufnahmedorn mag aus einem wärmebeständigen Kunststoff gefertigt sein.

Zur Verbindung des Grundkörpers mit dem den dampfleitenden Anschlußschlauch ist am Boden des Grundkörpers vorzugsweise ein, die Isolierstoffhülse durchdringender Anschlußstutzen angeordnet, mit dem der Anschlußschlauch dampfdicht verbunden ist. Der Anschlußschlauch besteht ebenfalls aus einem wärmebeständigen Kunststoff. Die Dimension des Anschlußschlauches in Durchmesser und Länge wird in Abhängigkeit vom Sterilisationsprozeß und verwendeten Chemo-Indikator angepaßt.

Die Erfindung ist in einem Ausführungsbeispiel auf der Zeichnung dargestellt und wird im folgenden näher beschrieben. Es zeigen:
- Fig. 1: den in einem Längsschnitt dargestellten Prüfkörper mit einem an seinem Anschlußstutzen befestigten Anschlußschlauch,
- Fig. 2: den in den Innenraum des Prüfkörpers einzuschiebenden Chemo-Indikator in perspektivischer Darstellung bei aufgeklappten Abdeckstreifen.

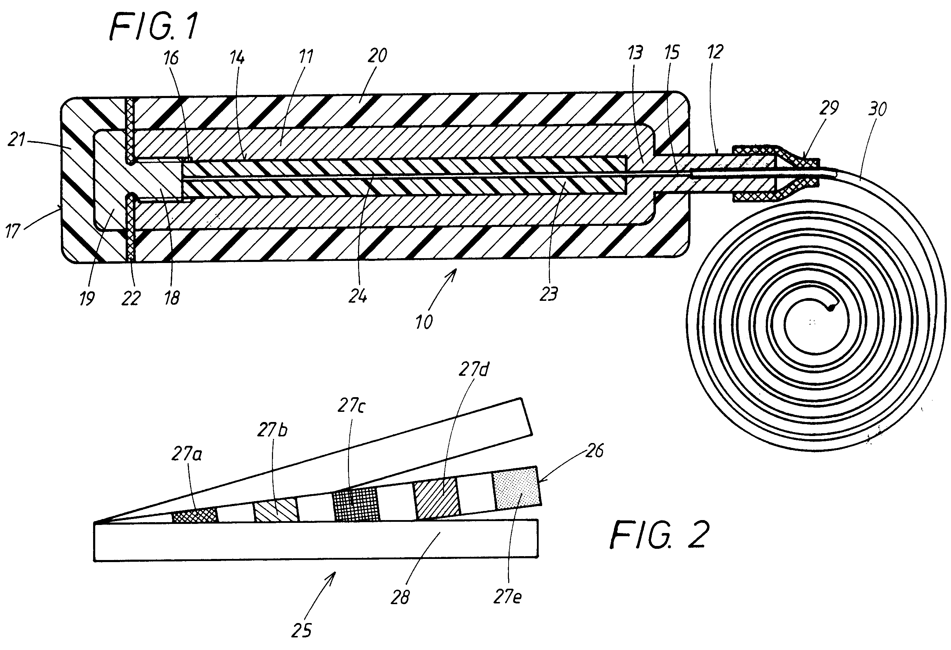

Der aus Fig. 1 ersichtliche Prüfkörper 10 weist einen etwa zylindrischen, hülsenartigen Grundkörper 11 auf, der aus Metall bestehend, dickwandig ausgebildet ist. An einem stirnseitigen Ende des Grundkörpers 11 befindet sich ein Anschlußstutzen 12, in dem sich eine auch den Boden 13 des Grundkörpers 11 durchdringende und zu dessen Innenraum 14 führende Bohrung 15 befindet. Ausgehend von der dem Anschlußstutzen 12 gegenüberliegenden Stirnseite befindet sich am Beginn des Innenraumes 14 ein Innengewinde 16 zur Aufnahme des Gewindezapfens 18 einer schraubbaren Kappe 17, mit welcher der Innenraum 14 des Grundkörpers 11 verschließbar ist. Auch die Schraubkappe 17 weist einen dickwandigen, scheibenförmigen Kern 19 auf, an den sich der Gewindezapfen 18 anschließt. Der Kern 19 und der Gewindezapfen 18 bestehen ebenfalls, wie der Grundkörper 11, aus Metall.

Der Grundkörper 11 und der Kern 19 der Schraubkappe 17 sind von einem Isolierstoffmantel umgeben, der entgegen der aus Fig. 1 ersichtlichen Darstellung auch aus zwei in Lägsrichtung getrennten Halbschalen bestehen könnte, die aus einem wärmedämmenden Kunststoff an einer Seite über ein Filmscharnier miteinander verbunden sein könnten und die auf der anderen Seite beispielsweise durch eine lösbare Rastverbindung zusammenzuhalten wären.

Bei dem aus Fig. 1 ersichtlichen Ausführungsbeispiel ist der Grundkörper außer auf seiner, die Öffnung des Innenraumes 14 aufweisenden Stirnseite, von einer Isolierstoffhülse 20 umgeben, die aus einem schwer wärmedurchlässigen Kunststoff gebildet sein mag und beispielsweise durch Umspritzen fest mit dem Grundkörper verbunden ist. Auch die Schraubkappe 17 weist eine den Kern 19, außer auf seiner den Gewindezapfen 18 aufweisenden Seite, umgebende Isolierstoffkappe 21 auf, die hinsichtlich Querschnitt und Werkstoffwahl der Isolierstoffhülse 20 entsprechen mag. Zwischen der Schraubkappe 17 und dem Grundkörper 11, mit der diesen umgebenden Isolierstoffhülse 20, ist eine Dichtung 22 angeordnet, deren Innendurchmesser den Gewindezapfen 18 umschließt, während ihr Außendurchmesser dem Außendurchmesser von Isolierstoffhülse 20 und Isolierstoffkappe 21 entspricht.

Im Gebrauchszustand des Prüfkörpers befindet sich im Inneraum 14 des Grundkörpers 11 ein Aufnahmedorn 23, in dem sich ein Längsschlitz 24 befindet, in welchen ein beispielsweise in Fig. 2 dargestellter Chemo-Indikator 25 eingelegt ist. Dieser Chemo-Indikator 25 kann im einfachsten Fall aus einer Trägerlamelle 26 gebildet sein, auf welchem sich mehrere unter gleichen Gegebenheiten umschlagende Indikator-Plättchen 27 befinden. Bei dem dargestellten Ausführungsbeispiel sind fünf Indikatorplättchen 27 a bis 27 e vorgesehen, die jedoch bei verschiedenen Temperaturen umschlagen.

Obschon nicht zwingend erforderlich, ist die Trägerlamelle 26 zumindest auf einer Seite, im aus Fig. 2 ersichtlichen Ausführungsbeispiel beiderseits von einem aufgeklappt dargestellten Abdeckstreifen 28 überdeckt. Dieser Abdeckstreifen besteht aus einem saugfähigen Werkstoff der beispielsweise ein saugfähiges Papier oder aber auch ein Vliesstoff sein kann.

In die Bohrung 15 im Anschlußstutzen 12 ist ein Anschlußschlauch 30 eingesteckt. Dabei mag die Anschlußstelle zwischen Anschlußstutzen 12 und Anschlußschlauch 30 mit Silikongummi 29 überzogen sein. Der Anschlußschlauch 30 selbst ist geringvolumig ausgebildet und weist nur eine geringe lichte Weite von einem bis wenigen Millimetern auf und kann in variabler Länge gestaltet werden. Der Werkstoff des Anschlußschlauches mag aus einem wärmebeständigen Kunststoff bestehen, wie er beispielsweise unter dem Handelsnamen Teflon am Markt erhältlich ist. Dabei kann der Innendurchmesser dieses Anschlußschlauches, je nach Anwendungsfall, zwischen einem und vier Millimetern gewählt werden. Entsprechend dem Anwendungsfall wird die Schlauchlänge zwischen 0,5 m und 6 m variieren können. Als Isolierwerkstoff für die Isolierstoffhülse 20 und die Isolierstoffkappe 21 können schlecht leitende Kunst-stoffe, geschäumte Kunststoffe oder andersartige wärmedämmende Stoffe Verwendung finden.

Die Dichtung 22 ist als Flachdichtung in Form einer Scheibe aus einem temperaturbeständigen, dauerelastischen Werkstoff gebildet, während der aus einem, mit einem Längsschlitz versehenen, zylindrischen Körper gebildete Aufnahmedorn, für den Chemo-Indikator aus einem wärmebeständigen Kunststoff gebildet sein kann, wie er beispielsweise auch für den Anschlußschlauch 30 Verwendung findet.

Die Trägerlamelle 26 des Chemo-Indikators 25 kann zu Dokumentationszwecken zusätzlich mit einem Klebestreifen versehen sein, der alle wesentlichen Daten enthalten kann, die für das Sterilisationsverfahren von Bedeutung sind.

Thermisch ansprechende Indikatorpapiere sind auf dem Markt in großer Zahl vorhanden. Es versteht sich jedoch, daß das verwendete Thermo-Indikatorpapier in bezug auf Temperatur und Zeit auf den jeweils zu prüfenden Sterilisationsprozeß abzustellen ist.

Wie bereits erwähnt gibt der dargestellte und vorbeschriebene Prüfkörper den Erfindungsgegenstand nur beispielsweise wieder, der keinesfalls allein darauf beschränkt ist. Es sind vielmehr noch mancherlei Änderungen und andere Ausgestaltungen der Erfindung denkbar. Außerdem sind alle in der Beschreibung erwähnten und/oder in der Zeichnung dargestellten, neuen Merkmale erfindungswesentlich, auch wenn sie in den Ansprüchen nicht ausdrücklich beansprucht sind.

| **Bezugszeichenliste** | |
|---|---|
| 10 Prüfkörper | 26 Trägerlamelle |
| 11 Grundkörper | 27a Indikatorplättchen |
| 12 Anschlußstutzen, an 11 | 27b Indikatorplättchen |
| 13 Boden, von 11 | 27c Indikatorplättchen |
| 14 Innenraum, von 11 | 27d Indikatorplättchen |
| 15 Bohrung, in 12 | 27e Indikatorplättchen |
| 16 Innengewinde, an 14 | 28 Abdeckstreifen |
| 17 Kappe, schraubbar | 29 Silikongummi |
| 18 Gewindezapfen, von 17 | 30 Anschlußschlauch |
| 19 Kern, von 17 | |
| 20 Isolierstoffhülse, von 11 | |
| 21 Isolierstoffkappe, von 19 | |
| 22 Dichtung | |
| 23 Aufnahmedorn | |
| 24 Längsschlitz, in 23 | |
| 25 Chemo-Indikator | |

## Patentansprüche

1. Prüfkörpersystem zum Überprüfen der Luftentfernung und/oder Sterilisationskontrolle kompletter Chargen in Dampfsterilisatoren, bestehend aus einem einen Anschlußschlauch aufweisenden Grundkörper mit einem darin angeordneten Aufnahmeschlitz, in dem sich ein Chemo-Indikator befindet und aus einer das freie Ende des Grundkörpers lösbar verschließenden Kappe,
**dadurch gekennzeichnet,**
daß der Grundkörper (11) und die Kappe (17) für eine hohe Wärmekapazität ausgebildet und im zusammengefügten Zustand allseits von einem Isolierstoffmantel (20, 21) umgeben sind.

2. Prüfkörpersystem nach Anspruch 1, dadurch gekenzeichnet, daß der Grundkörper (11) und die insbesondere schraubbare Kappe (17) aus Metall bestehen und der Grundkörper (11) von einer fest mit diesem verbundenen Isolierstoffhülse (20) umgeben ist, und auch die Kappe (17) in fester Verbindung mit einer Isolierstoffkappe (21) steht.

3. Prüfkörpersystem nach Anspruch 1 und/oder 2, dadurch gekennzeichnet, daß auf der Trägerlamelle (26) mehrere, jeweils bei unterschiedlichen Temperaturen umschlagende Indikatorplättchen (27a, 27b, 27c, 27d, 27e) angeordnet sind.
